# EUROPEAN PATENT SPECIFICATION

(11) **EP 2 617 451 B1**
(45) Date of publication and mention of the grant of the patent: **13.03.2019**
(21) Application number: 12382013.6
(22) Date of filing: 17.01.2012
(51) Int. Cl.: A61M 15/00

(54) **Drug delivery assembly, and single dose inhaler for dry powder drug delivery having such assembly**
Arzneimittelabgabeanordnung und Einzeldosisinhalierer zur Abgabe eines Trockenpulverarzneimittels mit solch einer Anordnung
Ensemble d'administration de médicaments et inhalateur de dose unique pour l'administration de médicaments sous forme de poudre sèche utilisant ledit ensemble

(43) Date of publication of application: 24.07.2013
(73) Proprietor: Laboratorios Liconsa, S.A., 08028 Barcelona (ES)
(72) Inventor: Hernández Herrero, Gonzalo, 28050 Madrid (ES); Andrade Benítez, Laura, 28009 Madrid (ES); Castellucci, Alessandro, 28002 Madrid (ES); Ronchi, Celestino, 20124 Milano (IT); Citterio, Mauro, 23807 Merate (LC) (IT); Lorenzo Gutiérrez, Isabel, 19200 Azuqueca de Henares (ES)
(74) Representative: ZBM Patents - Zea, Barlocci & Markvardsen

(56) References cited:
- WO-A1-96/32150
- WO-A2-02/089880
- WO-A2-2008/051471
- US-A- 3 991 761
- US-A- 5 699 789
- US-B2- 7 708 011

## Description

A drug delivery assembly for inhalers as defined in the preamble of claim 1 is herein disclosed. A single dose inhaler for dry powder drug delivery to a patient as defined in the preamble of claim 8 is also herein disclosed.

### BACKGROUND ART

Inhalers are currently widely used for the delivery of medication to a patient, such as for example for therapeutic treatment of the lungs. For example, in the treatment of asthma, inhalers are commonly used for delivering bronchodilators and anti-inflammatory agents. Other respiratory diseases such as bronchitis, emphysema, chronic obstructive pulmonary disease (COPD), etc. are also widely treated through the use of inhalers.

Dry powder inhalers (DPIs) are a particular type of inhalers that are also widely used for delivering both locally-acting and systemic drugs to the lungs. Drugs are delivered in the form of a dry powder. One of the most important advantages of the DPIs is that they do not make use of propellants. DPIs have also the advantage that they can deliver high drug doses. They also ensure drug stability and provide high patient compliance. These advantages make the DPIs to be more effective, reliable and safe than other inhalers such as metered dose inhalers (MDIs).

DPIs are often classified into single and multidose dose inhalers. Single dose inhalers make use of capsules, or blister packs, for predetermining the amount of the medication to be available with each inhalation. Multidose inhalers make use of a mechanism for measuring the dose for each inhalation. The present disclosure will be particularly referred to single dose DPIs.

Within the field of the dry powder inhalers it is known that the performance of such inhalers mainly depends upon the design of the inhaler itself, the powder formulation to be delivered by the inhaler and the airflow generated by the patient when using the inhaler. In this respect, many efforts have been made so far in improving the performance of DPIs. Such efforts have been However, no extensive research and development has been conducted towards the design features of dry powder inhalers themselves in order to improve performance.

The energy required for the delivery of drug particles to a patient is provided by the inspirational flow rate of the patient and the airflow resistance of the inhaler. The airflow resistance in a DPI is a function of its design. Inhalers having a high airflow resistance require a much higher inspiratory flow rate than inhalers with a much less airflow resistance.

Document US2009241949 discloses a drug delivery assembly comprising a dry powder inhaler and at least one cartridge containing a pharmaceutical formulation comprising at least one active ingredient for delivery to the pulmonary circulation. This document discloses that resistance values of the inhaler can be varied by changing the geometries or configuration of the air conduits so that airflow distribution through the cartridge and around the cartridge can vary. Inhalers can be structurally configured to have tunable airflow resistance by varying the cross-sectional area at any section of the airflow pathway of the inhaler and cartridge system. This document does not however teach any particular means or structure for adapting the required airflow for a suitable delivery of medication to a patient.

US3991761 discloses another example of an inhaler for the administration of powdered medicaments. It comprises a body portion, a mouth-piece with an inhalation passage and pivotably connected to the body portion, and a cap which covers the mouth-piece and rests against two pushbuttons. In use, the mouthpiece is rotated to expose a capsule recess so that a capsule is dropped therein. As the pushbuttons are depressed the ends of the capsule in the capsule recess are perforated.

WO02089680 shows an inhaler for producing an inhalable aerosol of a powdered medicament. It includes a vortex chamber having an inlet and exit ports. The ratio of the diameter of the vortex chamber to the diameter of the exit port is 4-12 while the length of the exit port is less than its diameter. An inlet conduit for supplying the medicament to the inlet port is provided whose cross-sectional area decreases in the direction towards the vortex chamber.

Therefore the problem still remains for an inhaler assembly that can be easily and efficiently adapted to the particular requirements of a patient in terms of airflow resistance for common drug dosages in DPIs.

### SUMMARY

A drug delivery assembly for inhalers is herein disclosed as defined in claim 1. The present drug delivery assembly is suitable for being fitted into any standard dry powder inhaler (DPI), although it is particularly suitable for being fitted into a single dose inhaler for dry powder drug delivery as defined in claim 8. Preferred embodiments are defined in the dependent claims.

The present drug delivery assembly comprises a body having a drug-receiving chamber formed at a bottom portion thereof. The drug-receiving chamber is suitably sized and shaped for receiving a medication capsule. The medication chamber is suitably sized and shaped for receiving a medication capsule. The medication capsule is of the type containing a drug in powder form for single dose delivery. An inhaler fitted with the present drug delivery assembly is particularly suitable for the delivery of a fluticasone propionate and Salmeterol combination.

The present drug delivery assembly further includes a fluid passage that is formed within the body. The drug-receiving chamber is in fluid communication with the fluid passage. A fluid flow path is defined through the fluid passage when air passes into the drug-receiving chamber. The fluid passage has at least one inlet portion through which air enters the body from the outside into the drug-receiving chamber. In some examples of the present drug delivery assembly, the fluid passage has two inlet portions provided at two opposite sides of the body. Outside air is allowed to enter the body through said opposite inlet portions into the drug-receiving chamber.

The inlet portion of the fluid passage is provided with at least one shaped protrusion. Where more than one inlet portions are provided, at least some of them may be provided with shaped protrusions, or even all of the inlet portions may be provided with shaped protrusions. An example of the present drug delivery assembly is, as stated above, with two inlet portions provided at two opposite sides of the body each having one shaped protrusion.

The shaped protrusions are in the form of tabs comprising a first segment and a second segment forming an angle to each other. In some examples, the first segment and the second segment of at least some of the shaped protrusions are arranged such that they both define substantially L-shaped protrusions. However, in some examples of the shaped protrusions the angle formed by the first segment and the second segment may be less than 90°.

The shaped protrusions are arranged protruding into the inlet portion of the fluid passage resulting in that the width of the fluid passage in the body is reduced up to 2.0 to 2.6 mm. Some examples provides a fluid passage having a width for the airflow of 2.4 mm.

The particular configuration of the shaped protrusions or tabs formed in the inlet portion or portions of the fluid passage with the inclined segments may result in that the flow of fluid passing through the fluid passage into the drug-receiving chamber is a substantially laminar flow. By reducing the likelihood of turbulent airflows in the fluid passage drug particles are prevented from coming into contact with the walls of the drug delivery assembly body and therefore from becoming adhered on inside surfaces. Laminar flow can be further ensured by a particular positioning of the protrusions or tabs relative to the fluid flow path in the inlet portion of the fluid passage. In particular, it is preferred that the second segment of at least some of the shaped protrusion is formed substantially parallel to the fluid flow path.

In some embodiments of the present drug delivery assembly, the first segment of at least some of the shaped protrusions may be formed integral with the body of the drug delivery assembly. However, in other examples, the first segment of the shaped protrusion may be a removable piece that can be detached from the body of the drug delivery assembly. This could be advantageous to rapidly adapt the inhaler where the present drug delivery assembly is fitted to a particular patient. This can be done by merely providing suitable protrusions or tabs in the fluid passage in order to provide the patient with a suitable drug delivery according to the particular patient's conditions.

In all of the above examples, the configuration of the protrusions or tabs may be suitably adapted as required. For example, in some embodiments the present drug delivery assembly it can be desirable that both the first and the second segments of the shaped protrusions or tabs are substantially of the same length.

It has been found that with the above drug delivery assembly the flow rate induced by the patient's inhalation strength adequately provides a given drug formulation according to the patient's particular requirements. The particular structure of the present drug delivery assembly and specifically that of the fluid passage into the drug-receiving chamber with the above shaped protrusions or tabs allow the required inspiratory effort by the patient to be easily controlled. Accurate clinically relevant inspiratory flow rates can be easily obtained for each inhaler.

The airflow flowing through the fluid passage encounters the shaped protrusions or tabs resulting in a suitable airflow resistance depending on the thickness of the protrusions and therefore on the width of the fluid passage (from 2.0 to 2.6 mm, as stated above) that causes the flow of fluid to the chamber to be a laminar flow. This is achieved by means of a very simple structure.

An inhaler fitted with the present drug delivery assembly is particularly suitable for the delivery of a fluticasone propionate and Salmeterol combination, as stated above. In one example, the drug may be presented as follows:
- Fluticasone Propionate/Salmeterol 100/50 µg DPI Product
- Fluticasone Propionate/Salmeterol 250/50 µg DPI Product
- Fluticasone Propionate/Salmeterol 500/50 µg DPI Product.

Salmeterol is presented as salmeterol xinafoate, where salmeterol base (50 µg) is equivalent to 72.5 µg salmeterol xinafoate salt.

Fluticasone Propionate (Fluticasone)/Salmeterol 100/50 µg, 250/50 µg and 500/50 µg Dry Powder Inhalation (DPI) product consists of a capsule dosage form containing a dry powder formulation of Fluticasone and Salmeterol for oral inhalation with the Monodose Inhaler (Monodose Inhaler 2.4 mm air path).

For the fluticasone/Salmeterol 100/50 µg DPI product, a clear Hydroxypropyl Methyl Cellulose (HPMC) capsule is provided containing a dry powder blend of 100 µg of Fluticasone Propionate and 50 µg of Salmeterol, active pharmaceutical ingredients (API) and up to 12.5 mg lactose monohydrate (lactose) as a carrier. Table 1 shows the formulation for Fluticasone/Salmeterol 100/50 µg DPI product.

**Table 1 Formulation for Fluticasone/Salmeterol 100/50 µg DPI product**

| Product | Materials | Functionality | Quantitative Composition |
|---|---|---|---|
| Fluticasone/ Salmeterol 100/50 µg DPI | Fluticasone Propionate | Active Pharmaceutical Ingredient | 100 µg |
| | Salmeterol Xinafoate | Active Pharmaceutical Ingredient | 72.5 µg ⁽¹⁾ |
| | Lactose monohydrate | Carrier, diluent | to 12.5 mg |
| | Size 3 Clear HPMC Capsule | Primary packaging | 1 capsule |

| | | | |
|---|---|---|---|
| ⁽¹⁾ 72.5 µg of Salmeterol xinafoate (equivalent to 50 µg of Salmeterol base). | | | |

For the fluticasone/Salmeterol 250/50 µg DPI product, a clear Hydroxypropyl Methyl Cellulose (HPMC) capsule is provided containing a dry powder blend of 250 µg of Fluticasone Propionate and 50 µg of Salmeterol, active pharmaceutical ingredients (API) and up to 12.5 mg lactose monohydrate (lactose) as a carrier. Table 2 shows the formulation for Fluticasone/Salmeterol 250/50 µg DPI product.

**Table 2 Formulation for Fluticasone/Salmeterol 250/50 µg DPI product**

| Product | Materials | Functionality | Quantitative Composition |
|---|---|---|---|
| Fluticasone/ Salmeterol 250/50 µg DPI | Fluticasone Propionate | Active Pharmaceutical Ingredient | 250 µg |
| | Salmeterol Xinafoate | Active Pharmaceutical Ingredient | 72.5 µg ⁽¹⁾ |
| | Lactose monohydrate | Carrier, diluent | to 12.5 mg |
| | Size 3 Clear HPMC Capsule | Primary packaging | 1 capsule |

| | | | |
|---|---|---|---|
| ⁽¹⁾ 72.5 µg of Salmeterol xinafoate (equivalent to 50 µg of Salmeterol base). | | | |

For the fluticasone/Salmeterol 500/50 µg DPI product, a clear Hydroxypropyl Methyl Cellulose (HPMC) capsule contains a dry powder blend of 500 µg of Fluticasone Propionate and 50 µg of Salmeterol, active pharmaceutical ingredients (API) and up to 12.5 mg lactose monohydrate (lactose) as a carrier. Table 3 shows the formulation for Fluticasone/Salmeterol 500/50 µg DPI product.

**Table 3 Formulation for Fluticasone/Salmeterol 500/50 µg DPI product**

| Product | Materials | Functionality | Quantitative Composition |
|---|---|---|---|
| Fluticasone/ Salmeterol 500/50 µg DPI | Fluticasone Propionate | Active Pharmaceutical Ingredient | 500 µg |
| | Salmeterol Xinafoate | Active Pharmaceutical Ingredient | 72.5 µg ⁽¹⁾ |
| | Lactose monohydrate | Carrier, diluent | to 12.5 mg |
| | Size 3 Clear HPMC Capsule | Primary packaging | 1 capsule |

| | | | |
|---|---|---|---|
| ⁽¹⁾ 72.5 µg of Salmeterol xinafoate (equivalent to 50 µg of Salmeterol base). | | | |

A pharmaceutical assessment of the product after filling was conducted using the Next Generation Impactor (NGI). The aerodynamic particle size distribution data at 4 kpa pressure drop for Fluticasone/Salmeterol 100/50, 250/50 and 500/50 µg DPI is as follows:

| | | |
|---|---|---|
| 100/50 µg | FLUTICASONE | SALMETEROL |
| FPF (%) | 24.86 | 26.39 |
| 250/50 µg | FLUTICASONE | SALMETEROL |
| FPF (%) | 23,93 | 25.52 |
| 500/50 µg | FLUTICASONE | SALMETEROL |
| FPF (%) | 23.16 | 23.59 |

| | | |
|---|---|---|
| FPF is defined as the fraction of the aerosolized drug with particle size < 5 µm. | | |

A highly efficient drug delivery assembly for a dry powder inhaler can be obtained through the use of the present drug delivery assembly. This drug delivery assembly is cost effective as it is easy to manufacture.

### BRIEF DESCRIPTION OF THE DRAWINGS

Additional objects, advantages and features of embodiments of the present drug delivery assembly and the present single dose inhaler having such assembly will become apparent to those skilled in the art upon examination of the present description, or may be learned by its practice.

One particular example of the present drug delivery assembly will be described in the following by way of a non-limiting example, with reference to the appended drawings, in which:
Figure 1 is a top perspective view of one example of a drug delivery assembly;
Figure 2 is a top perspective view of the example of the drug delivery assembly in figure 1 viewed from another, different side of the assembly;
Figure 3 is a top plan view of the example of the drug delivery assembly in figures 1-2; and
Figure 4 is an enlarged view of the inlet portion of the fluid passage that diagrammatically shows the structure of one of the shaped protrusions or tabs.

### DETAILED DESCRIPTION OF EMBODIMENTS

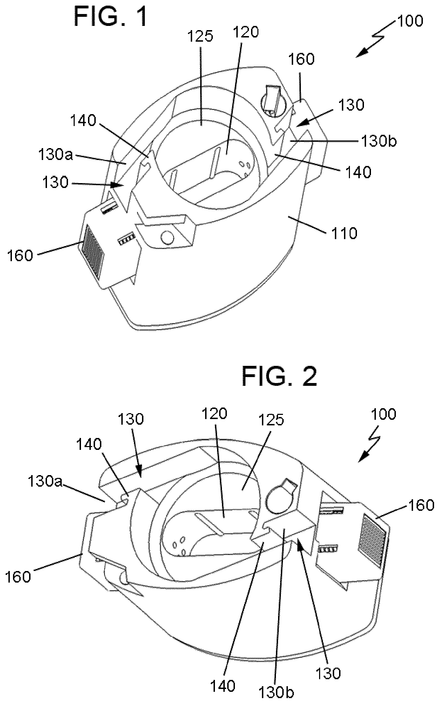

The figures 1-4 show a non-limiting example of the present drug delivery assembly. The drug delivery assembly has been indicated as a whole by reference numeral 100 throughout the present disclosure.

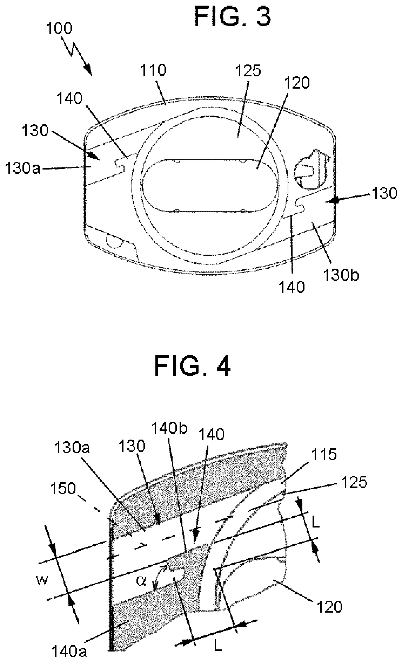

As shown in the figures, the drug delivery assembly 100 comprises a body 110. The body 110 has a central opening 115. Inside the opening 115 a drug-receiving chamber 120 is formed at a bottom portion 125 thereof. As shown in the top plan view in figure 3 of the drawings, the drug-receiving chamber 120 is shaped in the form of an elongated slot. The particular form and size of the chamber 120 is according to the medication capsule (not shown) to be received therein. Since the drug delivery assembly 100 shown in the figures and described as an example herein is for dry powder inhalators, the medication capsule is of the type containing a drug or medication in powder form for single dose delivery. One example of drug to be delivered with an inhaler fitted with the present drug delivery assembly 100 is a fluticasone propionate and Salmeterol combination.

The drug delivery assembly 100 according to the present invention further includes a fluid passage 130. The fluid passage 130 is formed within the body 110 of the drug delivery assembly 100 at an upper portion of the body 110 as shown in the figures. The fluid passage 130 in the body 110 of the drug delivery assembly 100 has a given width that will be described fully below and a suitable depth for the flow of an appropriate amount of air from the outside. The fluid passage 130 defines a fluid flow path 150 therein (path 150 depicted in dashed line in detail view of figure 4) when air passes from the outside into the drug-receiving chamber 120 upon inhalation by the patient.

The fluid passage 130 in the example shown has two opposite inlet portions 130a, 130b. The drug-receiving chamber 120 is formed between said two opposite inlet portions 130a, 130b of the fluid passage 120 such that the drug-receiving chamber 120 and the fluid passage 130 are in fluid communication to each other. Upon inhalation by the patient, outside air enters the drug-receiving chamber 120 via the two inlet portions 130a, 130b. A fluid comprising air and the formulation from the medication capsule received in the chamber 120 flows into an inhaler mouthpiece (not shown) in a direction that is substantially perpendicular to the plane of the drawing in figure 3 of the drawings.

As shown in the figures, the inlet portions 130a, 130b of the fluid passage 130 are both provided with one shaped protrusion 140. The shaped protrusions 140 are arranged such that the flow of fluid passing through the fluid passage 130 is a laminar flow. In the example shown, the shaped protrusions 140 are formed integral with the body 120 of the assembly 100. Referring now more particularly to figure 4, the shaped protrusions 140 each comprise a first segment 140a and a second segment 140b having substantially the same length (L). In this particular example, the protrusions 140 are formed as a unitary piece, that is, with the first and the second segments 140a, 140b formed integral with each other. Since the shaped protrusions 140 are formed integral with the body 120 of the assembly 100 as stated below, it is clear that the first segment 140a is therefore formed integral with the body 120 of the assembly 100.

The first and second segments 140a, 140b of the protrusions 140 form an angle α to each other, that is, they are substantially inclined to each other (not aligned). Referring again to figure 4, the angle α between the first and second segments 140a, 140b is substantially less than 90°. Other values of relative inclination of the segments 140a, 140b are of course possible as long as laminar flow is achieved. In addition, and as shown in said figure 4 of the drawings, the second segment 140b of the shaped protrusion 140 is formed substantially parallel to the fluid flow path 150 into the chamber 120.

Figures 3 and 4 show the segments 140a, 140b defining hook-like protrusions 140. The second segment 140b of the shaped protrusions 140 protrude each into the corresponding inlet portions 130a, 130b of the fluid passage 130. More particularly, the second segments 140b extend into the corresponding inlet portions 130a, 130b in the fluid passage 130 in a direction outwards the body 110. The provision of the second segment 140b into the corresponding inlet portions 130a, 130b of the fluid passage 130 results in the width (w) of the fluid passage 130 is reduced up to 2.4 mm, as shown in figure 4.

With the structure described, the operation of a single dose inhaler for dry powder drug delivery to a patient (not shown) comprising a drug delivery assembly 100 as described above is as follows. A cap (not shown) fitted on the mouthpiece (not shown) of the inhaler is first removed. Then, the mouthpiece is detached from the body 110 of the drug delivery assembly 100 such that the drug-receiving chamber 120 can be easily accessed. A medication capsule from, e.g. a blister strip with a number (e.g. 60) of pre-metered doses, can be then placed inside the chamber 120. The mouthpiece is then attached on the body 110 of the drug delivery assembly 100. Depending of the particular design of the inhaler, this can be carried by twisting the mouthpiece to the body 110. The user then squeezes the two opposite buttons 160 provided in the body 110 of the drug delivery assembly 100. This causes the medication capsule to be pierced and therefore the drug to be released to the chamber 120. At this moment, the user must breathe out fully and then insert the mouthpiece into his/her mouth and inhale quickly and deeply. The airflow caused by the inhalation by the patient through the fluid passage 130 along the flow path 150 encounters the shaped protrusions 140 resulting in an airflow resistance suitable for allowing a sufficient amount of the drug or active substance to be delivered into the lungs. After inhalation, the medication capsule can be discarded from the drug-receiving chamber 120.

Although only a number of particular examples of the present drug delivery assembly and the present single dose inhaler having such assembly have been disclosed herein, it will be understood by those skilled in the art that other alternative examples and/or uses and obvious modifications and equivalents thereof are possible. The claims cover all possible combinations of the particular embodiments described.

Reference signs related to drawings and placed in parentheses in a claim, are solely for attempting to increase the intelligibility of the claim, and shall not be construed as limiting the scope of the claim. Throughout the description and claims the word "comprise" and variations of the word, are not intended to exclude other technical features or components.

The scope of the present invention should not be limited by particular examples, but should be determined only by a fair reading of the claims that follow.

## Claims

1. Drug delivery assembly (100) for inhalers comprising a body (110) having a drug-receiving chamber (120) and a fluid passage (130) in communication thereto, the fluid passage (130) having at least one inlet portion (130a; 130b) through which a fluid flow path (150) is defined when fluid passes through the drug-receiving chamber (120), **characterized in that** at least one shaped protrusion (140) is formed in the inlet portion (130a; 130b) of the fluid passage (130), the shaped protrusion (140) comprising a first segment (140a) and a second segment (140b), arranged protruding into the inlet portion (130a; 130b) of the fluid passage (130) resulting **in that** the fluid passage in the body (110) has a width ranging from 2.0 to 2.6 mm and with the second segment (140b) of the shaped protrusion (140) being formed substantially parallel to the fluid flow path (150) such that the flow of fluid passing through the fluid passage (130) is a laminar flow.

2. Assembly (100) as claimed in claim 1, wherein the width of the fluid passage in the body (110) is 2.4 mm.

3. Assembly (100) as claimed in any of the preceding claims, wherein the first segment (140b) of the shaped protrusion (140) is formed integral with the body (110).

4. Assembly (100) as claimed in any of the preceding claims, wherein the first segment (140a) and the second segment (140b) of the shaped protrusion (140) forming an angle less than 90° to each other.

5. Assembly (100) as claimed in any of the preceding claims, wherein the first segment (140a) and the second segment (140b) of the shaped protrusion (140) are substantially of the same length.

6. Single dose inhaler for delivering a dry powder drug to a patient, the inhaler comprising a drug delivery assembly (100) as claimed in any of the preceding claims.

7. Inhaler as claimed in claim 6, wherein the drug comprises a fluticasone propionate and Salmeterol combination.

8. Inhaler as claimed in claim 7, wherein the drug is presented in the form of at least one of fluticasone propionate/Salmeterol 100/50 µg, 250/50 µg and 500/50 µg DPI product.

## Patentansprüche

1. Arzneimittelabgabeanordnung (100) für Inhalierer umfassend einen Körper (110), der eine Arzneimittelaufnahmekammer (120) und einen Flüssigkeitsdurchgang (130) in Verbindung damit umfasst, wobei der Flüssigkeitsdurchgang (130) mindestens einen Einlassteil (130a; 130b) umfasst, durch den ein Flüssigkeitsströmungsweg (150) definiert wird, wenn Flüssigkeit die Arzneimittelaufnahmekammer (120) durchgeht, **dadurch gekennzeichnet, dass** mindestens ein geformter Vorsprung (140) im Einlassteil (130a; 130b) des Flüssigkeitsdurchgangs (130) gebildet ist, wobei der geformte Vorsprung (140) einen ersten Abschnitt (140a) und einen zweiten Abschnitt (140b) umfasst, die derart angeordnet sind, dass sie in den Einlassteil (130a; 130b) des Flüssigkeitsdurchgangs (130) hinein ragen, wodurch der Flüssigkeitsdurchgang im Körper (110) eine Breite hat, die von 2,0 bis 2,6 mm reicht und wobei der zweite Abschnitt (140b) des geformten Vorsprungs (140) im Wesentlichen parallel zum Flüssigkeitsdurchgangsweg (150) gebildet ist, so dass die Strömung von der den Flüssigkeitsdurchgang (130) durchgehenden Flüssigkeit eine laminare Strömung ist.

2. Anordnung (100) wie in Anspruch 1 beansprucht, wobei die Breite des Flüssigkeitsdurchgangs im Körper (110) 2,4 mm beträgt.

3. Anordnung (100) wie in einem der vorhergehenden Ansprüche beansprucht, wobei der erste Abschnitt (140b) des geformten Vorsprungs (140) einstückig mit dem Körper (110) gebildet ist.

4. Anordnung (100) wie in einem der vorhergehenden Ansprüche beansprucht, wobei der erste Abschnitt (140a) und der zweite Abschnitt (140b) des geformten Vorsprungs (140) einen Winkel von weniger als 90° bezüglich einander bilden.

5. Anordnung (100) wie in einem der vorhergehenden Ansprüche beansprucht, wobei der erste Abschnitt (140a) und der zweite Abschnitt (140b) des geformten Vorsprungs (140) im Wesentlichen die gleiche Länge haben.

6. Einzeldosisinhalierer zur Abgabe von einem Trockenpulverarzneimittel an einen Patienten, wobei der Inhalierer eine Arzneimittelabgabeanordnung (100) wie in einem der vorhergehenden Ansprüche beansprucht umfasst.

7. Inhalierer wie in Anspruch 6 beansprucht, wobei das Arzneimittel eine Kombination aus Fluticasonpropionat und Salmeterol umfasst.

8. Inhalierer wie in Anspruch 7 beansprucht, wobei das Arzneimittel in Form von mindestens einem Pulverinhalatorprodukt aus Fluticasonpropionat/Salmeterol bei 100/50 µg, 250/50 µg und 500/50 µg dargeboten wird.

## Revendications

1. Ensemble d'administration de médicaments (100) pour des inhalateurs comprenant un corps (110) ayant une chambre de réception de médicaments (120) et un passage de fluide (130) en communication avec celle-ci, le passage de fluide (130) ayant au moins une partie d'entrée (130a ; 130b) à travers laquelle un chemin d'écoulement de fluide (150) est défini lorsque du fluide traverse la chambre de réception de médicaments (120), **caractérisé en ce qu'**au moins une saillie façonnée (140) est formée dans la partie d'entrée (130a ; 130b) du passage de fluide (130), la saillie façonnée (140) comprenant un premier segment (140a) et un second segment (140b), disposés en faisant saillie vers l'intérieur de la partie d'entrée (130a ; 130b) du passage de fluide (130) par lequel le passage de fluide dans le corps (110) a une largeur allant de 2,0 à 2,6 mm et le second segment (140b) de la saillie façonnée (140) étant formé essentiellement parallèle au chemin d'écoulement de fluide (150) de façon que l'écoulement de fluide traversant le passage de fluide (130) est un écoulement laminaire.

2. Ensemble (100) tel que revendiqué dans la revendication 1, dans lequel la largeur du passage de fluide dans le corps (110) est de 2,4 mm.

3. Ensemble (100) tel que revendiqué dans l'une quelconque des revendications précédentes, dans lequel le premier segment (140b) de la saillie façonnée (140) est formé de façon intégrale avec le corps (110).

4. Ensemble (100) tel que revendiqué dans l'une quelconque des revendications précédentes, dans lequel le premier segment (140a) et le second segment (140b) de la saillie façonnée (140) forment un angle inférieur à 90° l'un par rapport à l'autre.

5. Ensemble (100) tel que revendiqué dans l'une quelconque des revendications précédentes, dans lequel le premier segment (140a) et le second segment (140b) de la saillie façonnée (140) ont essentiellement la même longueur.

6. Inhalateur de dose unique pour l'administration d'un médicament sous forme de poudre sèche à un patient, l'inhalateur comprenant un ensemble d'administration de médicaments (100) tel que revendiqué dans l'une quelconque des revendications précédentes.

7. Inhalateur tel que revendiqué dans la revendication 6, dans lequel le médicament comprend une combinaison de propionate de fluticasone et salmétérol.

8. Inhalateur tel que revendiqué dans la revendication 7, dans lequel le médicament se présente sous la forme d'au moins un produit de type inhalateur de poudre sèche de propionate de fluticasone/salmétérol à 100/50 µg, 250/50 µg et 500/50 µg.
